# EUROPEAN PATENT APPLICATION

(11) **EP 4 053 111 A1**
(43) Date of publication of application: **07.09.2022**
(21) Application number: 21020112.5
(22) Date of filing: 01.03.2021
(51) Int. Cl.: C07D 301/12, C07D 303/42, C07C 67/00

(54) **THE METHOD OF PRODUCING EPOXIDISED RAPESEED OIL AND METHOD OF PRODUCING BIOPOLYOL USING EPOXIDISED RAPESEED OIL**

(71) Applicant: Komagra Spólka Z O.O., 01-303 Warszawa (PL)
(72) Inventor: Susik, Justyna, 43-100 Tychy (PL); Gloc, Monika, 41-218 Sosnowiec (PL); Sawicki, Grzegorz, 43-100 Tychy (PL)
(74) Representative: Karczmitowicz, Teresa Ewa

(57) **Abstract**

The method of producing biopolyol using purified epoxidized rapeseed oil produced by double bond epoxidation using an oxidation system consisting of dihydrogen peroxide 35% and glacial acetic acid 99% in the presence of an acid catalyst; purification by known methods; atmospheric distillation; removal of excess water; neutralization; rinsing the oil with hot water; vacuum distillation, characterized in that the epoxidized oil is subjected to a nucleophilic epoxy ring-opening process in the presence of an acid catalyst in a one-step or two-step epoxy ring-opening reaction using glycols or glycerol or methanol or fatty acid esters or water as the nucleophiles, followed by naturalization of the crude biopolyol with the catalyst; rinsed with water to obtain the acid number of 0.4 to 1.2 mgKOH/g; atmospheric steam distillation to obtain the acid number of less than 2 mgKOH/g; vacuum distillation to obtain a biopolyol with a water content of less than 0.1% m/m, iodine number of less than 10 gI2/100g; acid number of less than 2 mgKOH/g; and hydroxyl number of 80 - 320 mgKOH/g.

## Description

### FIELD OF THE INVENTION

The present invention relates to the method for producing epoxidised rapeseed oil intended as a starting material both for the production of various types of polyols and the method for producing a biopolyol using epoxidised rapeseed oil, particularly for the production of polyurethane plastics of various types and applications, e.g. flexible and rigid foams, but also foams for CASE applications .

### BACKGROUND OF THE INVENTION

The use of products of petrochemical origin for the production of polyols is well known from the literature. More recently, products of natural origin such as vegetable oils have also been used.

The Korean invention application No. KR20190099777 (A) relates to a method of producing biopolyols based on biomass, which are vegetable oils: soybean or castor oil as environmentally friendly components, with the aim of replacing conventional petroleum-derived polyols and thus reducing energy consumption and carbon dioxide emissions. The production of biopolyols involves the controlled introduction of ethylene oxide, propylene oxide or 1,3-propanediol into the biomass feedstock, thereby adjusting the molecular weight to between 1500 and 3000 um. The step of preparing the biomass feedstock includes the conduct of oxidation of the double bond of soybean oil with hydrogen peroxide to introduce an epoxy group and then carrying out the reaction with alcohol..

Korean Patent No. KR101631606 (B1) describes a method for producing a polyol by reacting epoxidised vegetable oil with a hydroxyl functional material, the reaction being carried out at a temperature between 50 °C and 200 °C under an alkali metal oxide catalyst, selected from MgO, CaO or a mixture thereof, the amount of which is in the range of 0.001 to 1% by weight per mass of epoxidised vegetable oil. The material with hydroxyl functional groups is water; C1-C6 aliphatic alcohols; polyhydric alcohols containing from 2 to 15 hydroxyl groups; and at least one selected from the group consisting of C2 to C10 alcohols. The material with hydroxyl functional groups is at least one selected from the group consisting of water, methanol, ethanol, ethylene glycol, pentaerythritol, ethanolamine, diethanolamine and triethanolamine. Epoxidised vegetable oil is produced by reacting hydrogen peroxide (H2O2) in the presence of an organic acid selected from among formic and acetic acids, after reacting methanol and sodium hydroxide with vegetable oil (rapeseed, soybean, sunflower, palm, etc.), and the epoxidation reactions are carried out to partially saturate the double bonds. Another Korean Patent No. KR101228570 (B1) discloses a method of producing a biopolyol for making rigid urethane foam from natural oil, comprising: a) an exchange step ofnatural reacting oils and a multifunctional active compound containing hydrogen in the presence of an alkaline catalyst at 70 to 120 °C for 1 to 3 hours. The exchange reaction involves 20 to 70% by weight of natural oil; b) reaction step of alkylene oxide addition to the exchange reaction product at 100 to 120 °C for 5 to 40 hours and, in addition, the step of catalyst removal from the product of the alkylene oxide compound addition reaction. Based on the total substrate weight of the biopolyol production reaction, at least 20% by weight of alkylene oxide is subjected to the addition reaction. Based on the total weight of the reactant, the preparation of the bio-polyol uses from 10 to 60% by weight of natural oil, from 15 to 50% by weight of a multifunctional compound containing active hydrogen ; from 20 to 70% by weight of alkylene oxide, the bio-polyol having the number of functional groups from 4 to 7, hydroxyl number from 300 to 600 mg KOH/g and viscosity (25 °C) of 2000 to 25000 cps.

The Chinese invention with the number CN100465152 (C) discloses a bio-polyol produced from epoxy rapeseed oil and a method of producing it comprising: carrying out a ring-opening reaction of epoxy rapeseed oil with an active nucleophilic reagent containing hydrogen in the presence of a catalyst to obtain a mixed hydroxy fatty acid triglyceride; adding an agent alcoholizing alcohols or alkanolamines and heating up to the alcoholysis reaction to obtain a mixed hydroxy fatty acid monoester, i.e. a biological polyol. The ring-opening reaction is carried out at a temperature in the range of 65 °C to 200 °C during 1 to 12 hours. The catalysts for the ring-opening reaction and the alcoholysis reaction are alkaline catalysts selected from metal hydroxides or metal alcoholates, prefereably, alkali metal hydroxides or alkali metal alcoholates, wherein the alkali metal hydroxides may be sodium hydroxide, potassium hydroxide, lithium hydroxide, etc.; the alkali metal alcoholates may be selected from sodium methanolate, sodium ethanolate, potassium methanolate, potassium glycerolate and other such alcohols and hydroxides.

European patent number EP2451857 (B1) describes a method for producing polyols comprising the steps of: (a) reacting natural unsaturated fats, natural unsaturated fatty acids and/or fatty acid esters with nitrous oxide, which may be used in a mixture with inert gases. ; (b) reacting the product obtained in step (a) with a hydrogenating reagent, which is complex metal hydride or lithium-aluminium hydride or sodium borohydride or lithium borohydride or hydrogen; (c) reacting the product of step (b) with alkylene oxides. Unsaturated natural fats as well as fat derivatives are selected from the group consisting of castor oil, grape seed oil, black cumin oil, pumpkin seed oil, borage seed oil, soybean oil, wheat germ oil, rapeseed oil, sunflower oil, peanut oil, apricot kernel oil, pistachio kernel oil, almond oil, olive oil, macadamia nut oil, avocado oil, sea buckthorn oil, sesame oil, hemp oil, hazelnut oil, evening primrose oil, rosehip oil, safflower oil, walnut oil, palm oil, fish oil, coconut oil, tall oil, corn germ oil, linseed oil. Fatty acids and fatty acid esters are selected from the group consisting of myristoleic acid, palmitic acid, oleic acid, wakenoic acid, petroselic acid, gadolic acid, erucic acid, nerve acid, linoleic acid, α- and γ-linolenic acid, stearidonic acid, arachidonic acid, thymnodonic acid, clupanodonic acid and cervonic acid and their esters. Unsaturated natural fats are selected from the group comprising soybean oil, palm oil, sunflower oil and rapeseed oil.

Preferably, step b) is carried out in the presence of a catalyst which contains at least one transition metal from groups 6 to 11 or in the presence of a catalyst containing ruthenium or nickel. The alkylene oxide addition reaction in step c) is carried out in the presence of a catalyst, preferably carried out in the presence of a multi-metal cyanide catalyst.

Chinese patent description No. CN105669450 (B) relates to a method for producing and using polyols from vegetable oil. A method for producing a polyol from vegetable oil having a high hydroxyl number, is characterized in that a polyhydroxyl and epoxy compound of the vegetable oil are reacted under the influence of a catalyst to produce a polyol, the reaction comprising a ring opening reaction between the hydroxyl group of the polyol and the epoxy group of the vegetable oil and a transesterification reaction between the hydroxyl group of the polyol and the ester bond of the vegetable oil. The polyhydroxy compound is the product of an addition reaction between a small-molecule alcohol and propylene oxide, wherein if the functionality of any of the small-molecule alcohols is n, the molar ratio to propylene oxide is 1: 1 to n. Conditions for the addition reaction: mixing of the small-molecule alcohol, propylene oxide and potassium hydroxide, followed by passing it under a nitrogen shield, mechanical stirring of the reaction at 30 °C for 5h, followed by neutralisation with phosphoric acid, dehydration and adsorption, and filtration through magnesium silicate. A polyhydroxy compound is obtained; wherein the mass percentage of potassium hydroxide and alcohol is 1%. According to the method, the epoxy vegetable oil is epoxy olive oil, epoxy peanut oil, epoxy rapeseed oil, epoxy cottonseed oil, epoxy soybean oil, epoxy coconut oil, epoxy palm oil, epoxy sesame oil, epoxy sunflower oil, epoxy linseed oil, epoxy castor oil, epoxy tung oil, epoxy rice bran oil and epoxy corn oil in one or a combination of several. The catalyst is sodium hydroxide, potassium hydroxide, sodium methanolate, sodium ethanolate, sodium isopropanolate, sodium n-butanolate, sodium tert-butanolate, potassium methanolate, potassium ethanolate, potassium isopropanolate, potassium tert-butanolate, tetrabutyl titanate, p-toluenesulfonic acid, antimony trioxide or stannous octanoate; wherein the mass percentage of the catalyst and the epoxy vegetable oil is 0.02-0.1%.

Chinese invention application No. CN110746299 (A) discloses a high quality polyol from vegetable oil and a method for its manufacture and use. A liquid mixture comprising hydrogen peroxide, an organic acid, a catalyst and a stabilizer, and a vegetable oil is pumped into a first microchannel microreactor of a modular reaction apparatus, respectively, to carry out an epoxidation reaction to obtain an epoxidised vegetable oil having an iodine number of 20-40, then the epoxidised vegetable oil and the cyclic compound, respectively, are pumped into a second microreactor of modular reaction apparatus and an initial ring-opening reaction is carried out to obtain the structural rigidity of the crude vegetable oil polyol, and finally the crude product and the small-molecule alcohol, respectively, are pumped into a third microreactor of a modular reaction apparatus with microchannels, to carry out a further ring-opening reaction to obtain a high-quality polyol. The poliol from vegetable oil polyol with a viscosity of 5000-6000 mPa. s is produced using microchannel technology and has moderate viscosity and good quality. Formic or acetic acid is used as the organic acid; sulphuric acid or phosphoric acid is used as the catalyst and ethylene dichloride is used as the stabiliser. Vegetable oil is soybean oil, corn oil, peanut oil, cottonseed oil, castor oil and sesame oil; the molar ratio of double bond, hydrogen peroxide, organic acid, catalyst and stabilizer in vegetable oil is 1: (0,83 - 1,06): (0,83 - 1,06): (0,005 - 0,01): (0,001 - 0,005).

U.S. invention application No. US2019119496 (A1) based on priority from Chinese invention application No. CN201811153270, discloses a method of producing a fully biological polyol from vegetable oil, involving enabling an epoxy vegetable oil to react with a compound of formula III in the second microstructured reactor to produce a polyol from vegetable oil.

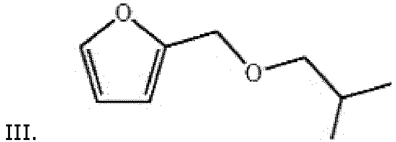

The method comprises the following steps: (1) simultaneously pumping a mixed solution of hydrogen peroxide, organic acid, catalyst and stabilizer and vegetable oil into a first microstructured reactor of a microchannel modular reaction device to obtain a reaction solution containing epoxidised vegetable oil; (2) simultaneously pumping the reaction solution containing the epoxidised vegetable oil obtained from step (1) and the compound of formula III into a second microstructured microchannel reactor of the modular reaction device to obtain a polyol from the vegetable oil.

The organic acid in the step (1) is formic acid or acetic acid, the catalyst is sulfuric acid or phosphoric acid, the stabilizer is ethylenediaminetetraacetic acid, the vegetable oil is at least one selected from olive oil, peanut oil, rapeseed oil, cottonseed oil, soybean oil, palm oil, sesame oil, sunflower oil, linseed oil, tung oil, safflower oil, rice oil, maize oil and coconut oil, and the molar ratio of double bonds in vegetable oil to hydrogen peroxide to organic acid to catalyst to stabiliser is 1: (6-20) : (6-20) : (0.02-0.4) : (0,006-0,2). The first microstructured reactor in the step (1) has a reaction temperature of 60-130° C, a reaction time of 5-10 minutes; the vegetable oil is pumped into the microchannel modular reaction device at a flow rate of 0.5-1.0 ml / min, and the mixed solution is pumped into the microchannel modular reaction device at a flow rate of 3.5-5.0 ml/min. The molar ratio of epoxy groups in the epoxy vegetable oil to the compound of formula III in the step (2) is 1: (1.5-4.5), the second microstructural reactor has at a reaction temperature of 70-100 ° C, a reaction residence time of 6-10 min and a volume of 96-240 ml, the compound of formula III is pumped into a microchannel modular reaction device with a flow rate equal to 12.0-18.0 ml/min. The compound of formula III in the step (2) is produced by the following method, comprising: (a) dissolving furfuryl alcohol in the reaction solvent, dropping thionyl chloride into the solution at -10 ° C to 10 ° C, continuing to stir and react for 0.5-2 h, adding water to stop the reaction, collecting the organic phase and vortex drying to obtain a colourless liquid; (b) then adding glycerol and sodium to the colourless liquid, continuing to stir and carrying out the reaction for 3-6 h at 30-50 ° C, with the formation of the compound of formula III.

In general, polyurethane plastics are produced using mainly polyols of petrochemical origin, with crude oil as the base material. For a few years now, polyol products have been appearing on the market, the production of which is increasingly based on a vegetable base, where the state of the art shows that in addition to the natural oil base, various chemical additives are also used to enable the proper process of polyol production.

The state of the art reveals that obtaining of polyols with hydroxyl number in the range of 80-320 mgKOH/g requires carrying out epoxidation process until the epoxide number reaches the value in the range of 0.15-0.35 mol/100g of epoxidised oil, not taking into account the height of iodine number, while a high iodine number, above 10 gI2/100g, indicates that more than 0.04 mole of double bonds per 100 g of epoxide is left in the epoxide. In time, the double bonds undergo free radical oxidation, initiated by the action of e.g. oxygen, thus affecting the durability of the polyol and, in turn, accelerated degradation of the polyurethane material itself.

In view of the above and taking into account the current state of the art, the inventor formulated an inventive problem, i.e. development of a method to produce epoxidised rapeseed oil, intended for the production of polyols, and in particular for the production of biopolyols, and a method to produce biopolyols using epoxidised rapeseed oil as a substitute for petrochemical polyol components used in the production of polyurethane

### BRIEF OF THE DESCRIPTION

The objective of the invention is a method for producing epoxidised rapeseed oil and a method for producing a biopolyol based on epoxidised rapeseed oil using an epoxidation process with reduced selectivity, leading to the saturation of as many double bonds as possible, i.e. to achieve an iodine number below 10 gI2/100g of oil.

The objective of the invention is the method for producing epoxidised rapeseed oil using the process of double bond epoxidation in the presence of a catalyst, steam distillation of the epoxidised oil and its dehydration using rapeseed oil as a base, obtained after pre-treatment comprising hot pressing followed by solvent extraction, degumming and neutralisation with phosphoric acid and deacidification with soda lye, and characterised in that the rapeseed oil having a free fatty acid content of 0,02 to 0,1 % m/m a phosphorus content of 5 to 100 ppm; a peroxide number of not more than 5 mqO2/kg; an alkalinity of 10 to 100 ppm; a water content of not more than 0.1 % m/m; an iodine number of 105 to 120 gI2/100g; an unsaponifiable matter content of not more than 1.5 % m/m, and a monoacylglycerol, diacylglycerol and free glycerol content of not more than 1 % m/m in total, is placed in a reactor equipped with a stirrer and subjected to: [1] the process of double bond epoxidation using an oxidation system consisting of dihydrogen peroxide 35%, which content in relation to each mole of rapeseed oil double bonds is within the limits from 1.0 to 5.0 moles and glacial acetic acid 99%, with a content for each mole of rapeseed oil double bonds is within the limits from 0.1 mole to 1.0 mole and in the presence of an acid catalyst in the amount of 1.0 to 6.0 g m/m of concentrated catalyst per 100 g m/m of reaction mixture and the epoxidation process is carried out at a stirrer speed of 800 to 1800 rpm at a temperature from 35 to 75°C for 5 to 16 h, with an epoxidation reaction selectivity of more than 70% and an epoxidation reaction conversion of more than 90% and after the epoxidation process is completed, the resulting epoxidised oil is subjected [2] to purification by known methods from the residues of dihydrogen peroxide 35% and acetic acid 99%; a [3] distillation process under atmospheric pressure using saturated steam until an acid value of less than 2 mg KOH/g, preferably less than 1 mg KOH/g, is achieved; [4] removal of excess water by centrifugation; [5] neutralisation using a 30% sodium methanolate solution in proportion to the amount of catalyst used in the epoxidation process of 3:1 /mole: mole/; [6] washing of the oil several times with hot water; [7] separation of the resulting mixture by a known method; [8] drying by vacuum distillation at 80-120°C using a vacuum of 0.6 - 0.8 mbar until a water content below 0.1% m/m is achieved, and [9] storage of the epoxidised rapeseed oil for further processing as required

The object of the invention is also the method of producing biopolyol using purified epoxidised oil produced by the above-described method in the process of epoxidation, atmospheric distillation with steam, centrifugation, neutralization, washing and vacuum distillation to obtain oil with a content of less than 0.1% by weight or using purified and centrifuged epoxidised oil produced by the above-described method in the process of epoxidation, atmospheric steam distillation and centrifugation to a water content of 2-5% m/m, followed by [1] subjecting the thus purified epoxidised oil to an epoxide ring-opening reaction with nucleophile in the presence of an acid catalyst, wherein the purified epoxidised oil having an epoxide number of 0.1-0.2 mol/100 grams of epoxidised oil being subjected to a one-step epoxide ring-opening reaction with nucleophile at a ratio of 1-2 mole of nucleophile to moles of epoxy groups of the purified epoxidised oil, with the participation of an acid catalyst in the amount of 2 to 5 g/1 mole of epoxy groups, and the purified epoxidised oil having an epoxy number of 0,29-0,35 mole/100 g of epoxidised oil is subjected to a two-step epoxy ring-opening reaction, wherein in the first step the reaction is carried out in the presence of water with a molar ratio of 5 to 10 moles, preferably 6 moles of water to moles of epoxy groups, and with the participation of an acid catalyst in the amount of 2 to 5 g/1 mole of epoxy groups, until an epoxide number in the range of 0.10 to 0.22 mol/100g of epoxidised oil is obtained, and in the second step, the reaction is carried out between the remaining epoxidised groups in the oil after the first step and a nucleophile in the molar ratio of 1-2 moles of nucleophile to moles of epoxy groups and with the participation of an acid catalyst in the amount of 2 to 5 g/1 mole of epoxy groups; and the biopolyol thus obtained is successively subjected [2] to a catalyst naturalization process; [3] to rinsing with water until an acid number from 0.4 to 1.2 mgKOH/g is obtained; [4] to distillation using saturated steam at atmospheric pressure until an acid number below 2 mgKOH/g is obtained; [5] to a vacuum distillation process under negative pressure of 0.5 - 1.0 mbar to obtain a biopolyol having the properties: iodine number below 10 gI2/100g, acid number below 1.2 mgKOH/g, hydroxyl number in the range 80 - 320 mgKOH/g.

### DETAILED OF THE DESCRIPTION

As a base for the production of purified epoxidised oil as well as biopolyol according to the invention, rapeseed oil was used, previously produced in the process of hot pressing and then subjected to the process of solvent extraction and then degumming and neutralization with phosphoric acid and sodium lye deacidification, carried out by known methods, in order to significantly reduce contaminants such as: alkalinity, phospholipids, free fatty acids and non-fatty substances. Preliminary processes cause improvement of organoleptic properties of the oil as well as reduction of impurities, which, as it turned out, has a significant influence on the quality of the epoxy obtained by the method according to the invention. Tests and trails have shown that the best properties are obtained for the polyol produced from degummed oil previously initially treated, which is characterised by the following properties:
- low content of free fatty acids in the range of 0.1 - 0.02% m/m, preferably 0.06 - 0.02% m/m;
- phosphorus content in the range from 5 to 100 ppm, preferably from 5 to 20 ppm;
- low oxidation state, expressed as peroxide number, of not more than 5 mqO2/kg, preferably not more than 0.5 mqO2/kg
- low alkalinity of between 10 and 100 ppm, preferably between 10 and 50 ppm
- low water content of not more than 0.1% m/m, preferably not more than 0.05% m/m;
- iodine number between 105 and 120 gI2/100g, preferably 110+/-2gI2/100g. The iodine number defines with high probability a double bond content of from 0.41 to 0.48 moles of unsaturated bonds in 100g of oil, more preferably 0.43 moles of unsaturated bonds in 100g of oil;
- maximum unsaponifiable matter content of 1,5 % m/m, and
- low content of monoacylglycerols, diacylglycerols and free glycerol, which together do not exceed 1% m/m.

Rapeseed oil with the properties indicated above was successively subjected to an epoxidation process. In the course of numerous trials and tests, it was observed that the alkalinity of the decomposed rapeseed oil, amounting to more than 100 ppm, causes a reduction of the catalyst activity in the epoxidation process and prevents proper purification of the epoxidised oil, while free fatty acids reduce the efficiency and selectivity of the epoxidation process. In the final stage of biopolyol production, during product purification, free fatty acids react with the neutraliser to form soaps and thus hinder product separation and purification. The tests have also shown that the higher the peroxide number in the epoxidation step, i.e. above 5 mqO2/kg, the more efficient the process is, as the amount of side-effect reactions also increases.

The epoxidation process is carried out in a fully metered reactor equipped with a mechanical stirrer with a stirring tip causing mixing in the entire liquid volume, e.g. a disc stirrer, while the use of a different stirrer does not limit the use of mixing, e.g. with a pump and dynamic or static mixers.

Degummed rapeseed oil is introduced into the reactor and the process of double bond epoxidation is carried out using an oxidising system: dihydrogen peroxide 35% and glacial acetic acid 99% in the presence of an acid catalyst, e.g. sulphuric acid VI or phosphoric acid V or hydrochloric acid or other inorganic acids. Studies and numerous laboratory and semi-technical trials have shown how important it is to maintain the proportions of the reactants and have determined the optimum content of acetic acid 99% in the process to be 0.1- 1.0 mole of acetic acid 99%, preferably 0.4 - 0.7 mole for each mole of unsaturated bonds in rapeseed oil, while the molar ratio of dihydrogen peroxide 35% to each mole of double bonds in rapeseed oil should be in the range of 1.0 - 5.0, preferably 1.5 - 2.0 moles. The proportion of catalyst used in the epoxidation process is in the range of 1.0 -6.0g m/m, preferably 2.0 -3.5g.m/m of concentrated catalyst per 100g m/m of reaction mixture.

The epoxidation process is carried out at a stirrer speed of 800-1800 rpm, preferably 1500 rpm, and a temperature of 35 - 75°C, preferably 63 °C. The initial stage of the reaction is an exothermic process, so it is important to add acetic acid 99% including the catalyst, as a single portion, and only then add dihydrogen peroxide 35%

Dihydrogen peroxide 35% is introduced into the reactor at a temperature of 35 - 55 °C, preferably 45 °C, by gradually dropping it in at a constant rate of 1 to 10 °C /1h, preferably 4 °C /1h, and at a constant rate of temperature increase so that all the oxidant is dispensed before the temperature reaches 55°C. After all components have been injected, the reaction mixture is heated gradually until the reaction temperature reaches 35 - 75°C, preferably 57 - 63 °C.

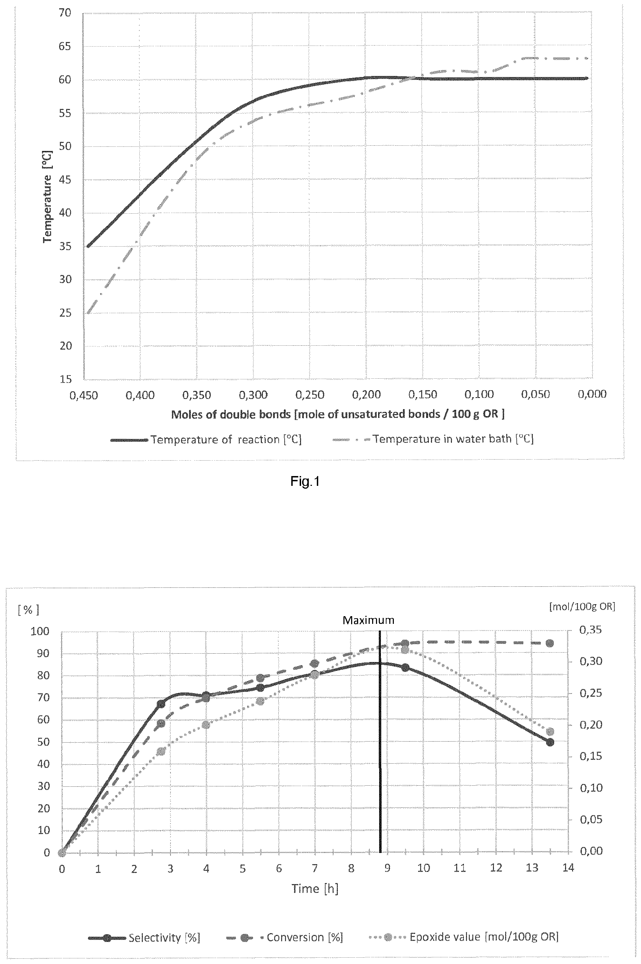

Throughout the process, the rate at which the oxidising agent is introduced into the reaction mixture is strictly controlled with respect to the temperature prevailing in the reactor. Numerous tests have revealed that when oxidant is added at temperatures below 35 °C, local overheating in the reactor can occur, causing a reduction in the selectivity of the process, while temperatures above 75 °C pose the risk of the reaction mixture reaching boiling point as a result of exothermic chemical reactions taking place in the reactor. As the reaction progresses, i.e. the double bonds are saturated, the exothermic effect decreases, therefore it is necessary to maintain the temperature of the reaction, thus maintaining its efficiency. This is done by adjusting the temperature of the water bath. The dependence of the reaction progress in relation to the reaction temperature and the water bath temperature has been illustrated in the diagram in **Fig. 1****.**

Moreover, both laboratory tests and semi-technical trials, in which the selectivity and conversion of the epoxidation reaction was continuously controlled, revealed that to optimise the process, the selectivity of the epoxidation reaction should be above 70%, preferably above 75%, while the conversion of the reaction should be above 90%, preferably above 95%.

The results of the study related to the dependence of the selectivity and conversion of the epoxidation reaction with respect to the obtained epoxide number value higher than 0.3 mol/100 g of oil, are presented in the diagram constituting **Fig.2****.**

An important parameter of the epoxidation process in connection with the selectivity and conversion of the reaction is also the epoxidation reaction time, the determination of which is always carried out during the process with the use of continuous monitoring of the reaction course and process conditions. As indicated above, an effective course of the process occurs when the conversion of unsaturated bonds to epoxy groups takes place with a selectivity of over 70% and a reaction conversion of over 90%. The reaction then has a point at which the newly formed epoxidised oil is converted to secondary products. The reaction time is determined by monitoring the reaction mixture for a decrease in iodine number and an increase in epoxidised oxygen content.

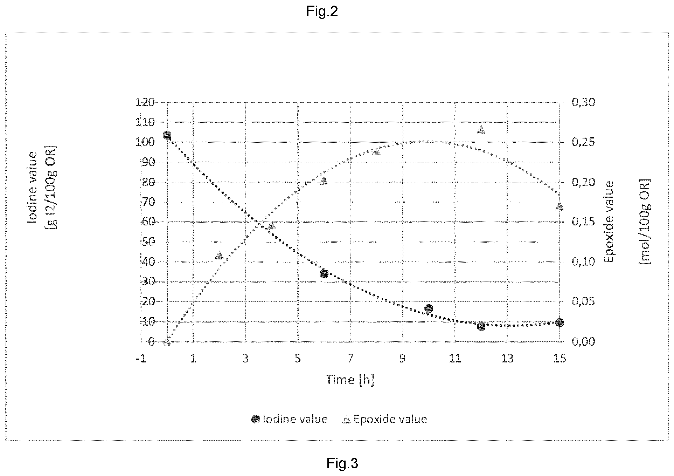

The dependence of iodine number and epoxide number with respect to reaction time is shown in the diagram in **Fig.3**

Exceeding the reaction time results in the involvement of secondary reactions, such as the opening of epoxy rings by means of factors contained in the reaction mixture, for example, water produced by peroxide consumption, acetic acid or catalyst incorporation. It should be pointed out that a decrease in the iodine number with a low increase in epoxidised oxygen indicates that the process is running at high efficiency with low selectivity, i.e. an increase in the proportion of undesired reactions that lead to the simultaneous opening of epoxy rings, most often with water or acetic acid. It is therefore essential to maintain a strict production regime.

Numerous trials and tests have shown that to obtain epoxidised oil with an epoxide number of 0.21-0.35 mole/100 g, the epoxidation process time is in the range of 5 to 16 h, preferably 9 h, where qualitative tests of the degummed rapeseed oil, which is the feedstock for the epoxidation process, are used to determine the precise end point of the reaction, especially the iodine number, which indicates the amount of unsaturated bonds. As a result of such determined process time, epoxidised oil is obtained.

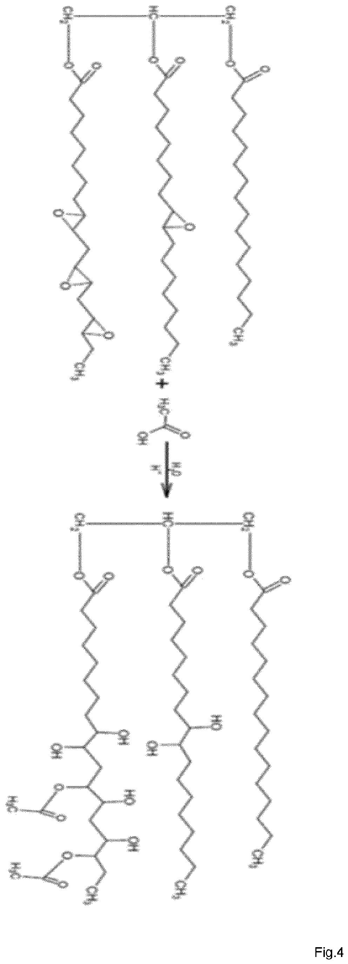

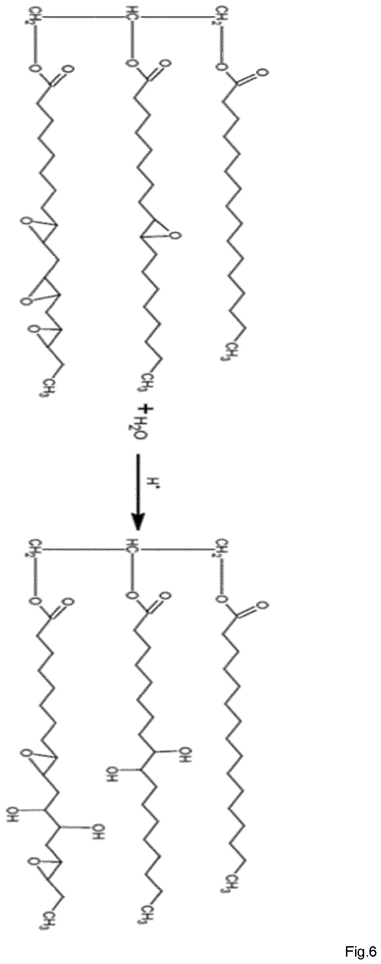

Tests have also revealed that obtaining an epoxide number value of 0.1 - 0.2 mol/100g of epoxidised oil, requires the epoxidation time to be extended from 16 to 20h, preferably 17-18h. This is due to the occurring secondary reactions, such as the reaction of acetic acid with the epoxide groups formed **(****Fig 4****)**, and/or the reaction of water with the epoxidised groups **(****Fig 6****).**

As a result of this process, an epoxidised oil with the following characteristics is obtained: epoxide number 0.1-0.2 mol/100g of epoxide oil, preferably 0.15 mol/100g; iodine number less than 10 gI2/100g, preferably less than 5 g I2/100g.

The other relevant properties of the epoxidised oil with the epoxide numbers indicated above are as follows:
- iodine number less than 10 gI2/100g, preferably less than 5 gI2/100g,
- acid number of less than 1 mgKOH/g, preferably less than 0.6 mgKOH/g;

After completion of the epoxidation process, the reactor contents are cooled to room temperature. The epoxidation process results in a two-phase mixture consisting of an oil phase containing reacted rapeseed oil, so-called crude epoxidised oil, and an aqueous phase containing water, unreacted dihydrogen peroxide, catalyst and acetic acid. The oil phase is separated from the aqueous phase and the crude epoxidised oil is purified by known methods from residual acetic acid and dihydrogen peroxide, the presence of which may significantly interfere with subsequent processes for producing the purified epoxidised oil and effectively also with processes leading to the production of biopolyol. For example, unseparated acetic acid behaves as an autocatalyst, which is very likely to cause the epoxy rings to open and thus reduce the selectivity of the reaction. Furthermore, acetic acid often undergoes side reactions, thus contaminating the intermediate product with substances such as hydroxyacetone or dihydroxyacetone, which may affect the crystallisation of the raw epoxy, which is an intermediate product for further processing.

The epoxy ring-opening reaction with acetic acid is illustrated in **Fig.4****.**

The crude epoxidised oil is distilled at atmospheric pressure (1 bar +/- 0.001 bar) using saturated steam to maintain the process temperature.
Laboratory tests and semi-technical trials have confirmed that distillation in the presence of steam allows the structure of the crude epoxidised oil to be preserved, so that the epoxidised oil does not mix with water and it is possible to separate volatile components by distilling them off with steam.

On completion of the distillation process in the presence of steam, epoxidised oil free of volatile organic compounds such as acetic acid and its transformation products is obtained, while the by-product of the distillation process is condensate containing water and organic impurities. The distillation process is carried out to achieve an acid number of the epoxidised oil of less than 2 mg KOH/g, preferably less than 1 mg KOH/g. The purified epoxidised oil obtained in the process has the following parameters:
- an epoxidised oxygen content of from 4.0 to 6.0% by weight, preferably above 5.0% by weight
- an iodine number of less than 10 gI2/100g, preferably less than 5 gI2/100g;
- an acid number of less than 1.0 mgKOH/g, preferably between 1.0 and 0.7 mgKOH/g;

After atmospheric steam distillation, the purified epoxidised oil contains up to 30 % m/m water, the excess of which is removed by centrifugation, e.g, using a centrifuge with a speed of 4500 - 5000 rpm, for 2 - 5 min, resulting in a cleaned and centrifuged epoxy oil with a water content of 2 - 5% m/m.

To allow the purified epoxy oil to be stored and become a commercially viable product, it is sent to a neutralisation and washing process to remove residual catalyst, which if left in the product results in its incorporation into the epoxy structure. On the other hand, leaving water above 0.1% m/m. in the stored epoxidised oil leads to the opening of some of the epoxy groups, causing a decrease in the epoxidised oxygen content and an increase in the hydroxyl number.

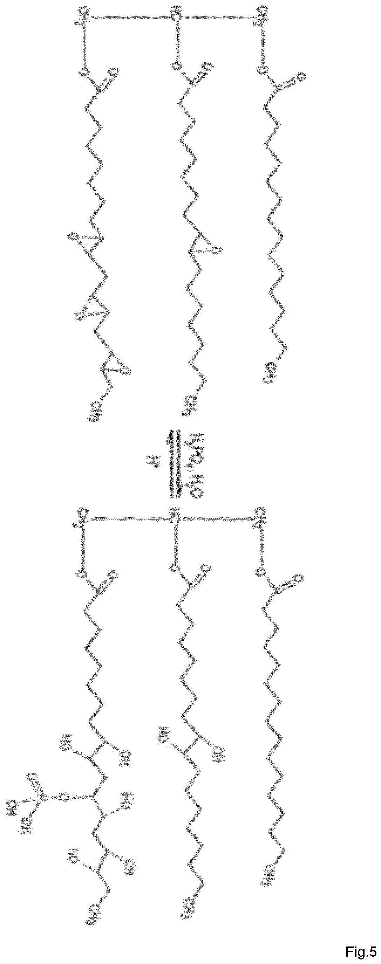

The side reaction of the incorporation of catalyst residues into the epoxide structure is shown in **Fig.5****,** while the reaction of epoxy ring opening due to the presence of water is shown in **Fig. 6****.**

Neutralisation is carried out using a 30% sodium methanolate solution in a stoichiometric amount relative to the amount of catalyst used in the epoxidation process and the acid number. When phosphoric acid V is used as a catalyst, the ratio of sodium methanolate to catalyst is 3:1 mol/mol. Subsequently, the epoxidised oil is washed several times with hot water and the resulting mixture is separated by a known means, e.g. by using a pear-shaped distributor with a tap, and then the epoxidised oil is dried using vacuum distillation, which is carried out at a temperature of 80-120°C, preferably 120°C using a vacuum of 0.6 - 0.8 mbar, preferably 0.8 mbar. The distillation process is carried out until a water content of less than 0,1% m/m, preferably less than 0.06% m/m, is obtained, yielding a dried, purified epoxidised oil which can be safely stored until it is used in the manufacture of various types of polyols.

The method of producing a biopolyol according to the invention is carried out using the purified and dried epoxidised oil produced by the above-described process of epoxidation, atmospheric distillation with steam, centrifugation to a water content of 2-5% m/m, subsequent neutralization, washing and vacuum distillation to obtain an oil with a water content of less than 0.1% m/m or using cleaned and centrifuged epoxidised oil produced by the above process of epoxidation, atmospheric steam distillation and centrifugation to a water content of 2-5% m/m, which immediately after centrifugation is passed to a further process, in which case the production of the bio-polyol is carried out in a continuous process, omitting the three processes necessary to enable the epoxidised oil to be stored without losing its physico-chemical properties, i.e. neutralisation, washing and distillation under vacuum.

The stored epoxidised oil or epoxidised oil diverted directly after centrifugation to a water content of 2-5% m/m is delivered into a reactor equipped with a stirrer and full metering, and subject to an epoxy ring-opening process by reaction of epoxy groups with a nucleophile, in the presence of an acid catalyst. During the reaction of the epoxy groups with nucleophile, crosslinking occurs between the free hydroxyl groups with the attached nucleophile and the remaining epoxy groups.

Glycols or glycerol or methanol or esters of higher fatty acids or water are used as nucleophiles.

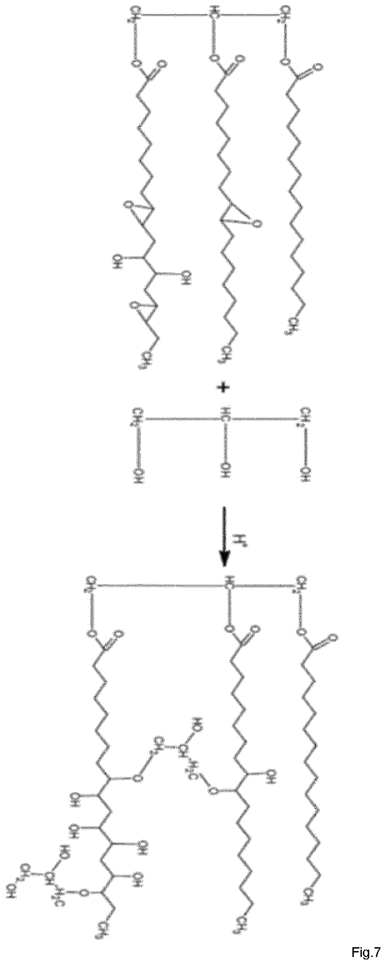

An example of a crosslinking reaction using glycerol is shown in **Fig. 7****.**

The method of producing a biopolyol can be carried out using either a one-step epoxy ring opening reaction using a single opening agent or a two-step epoxy ring opening reaction using two opening agents.

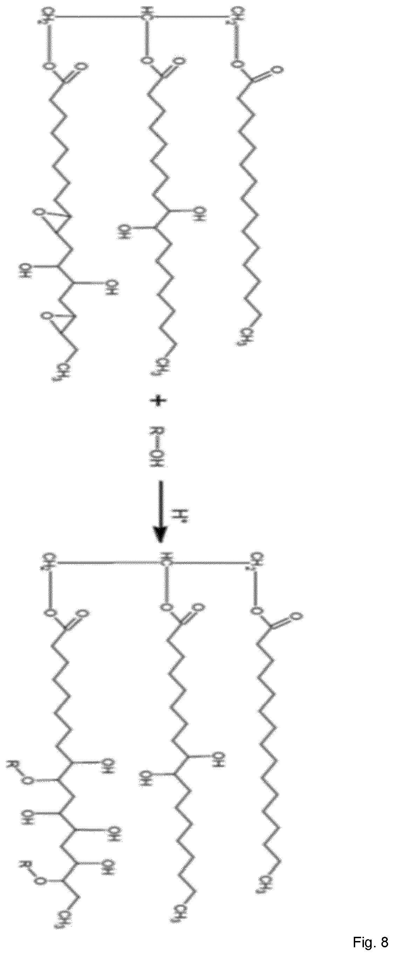

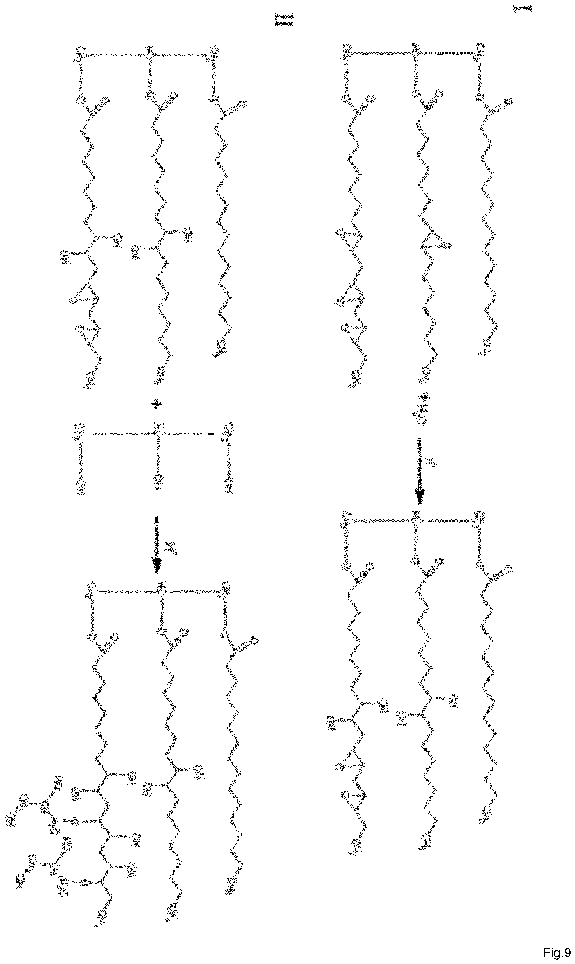

The epoxy group opening reaction in the one-step process using a single nucleophile is illustrated in **Fig. 8****,** while the epoxy ring-opening reactions in a two-step process, in which the first step is the epoxy ring-opening reaction with water and the second step with glycerol - are shown in **Fig. 9****.**

The one-step process is carried out for an epoxidised oil having an epoxide number of 0.1-0.2 mole/100 g of epoxidised oil, preferably 0.15 mol/100 g; iodine number of less than 10 gI2/100g, preferably less than 5 gI2/100g; and an acid number of less than 1 mgKOH/g, preferably 0.6 mgKOH/g. The oil is subject to the epoxy ring-opening reaction with nucleophile at a molar ratio of 1-2 moles of nucleophile to moles of epoxy groups, preferably 1.2 moles of nucleophile to moles of epoxy groups, in the presence of the acid catalyst in the amount of 2 - 5 g/1 mole of epoxy groups, preferably 2.5 - 3.5 g/ 1 mole.

Purified epoxidised oil is introduced into the mixture of catalyst and nucleophile, and the whole mixture is stirred at the stirring speed of 1000-1800 rpm, preferably not less than 1500 rpm, and heated to a reaction temperature of not less than 100 °C, preferably 120+/- 5 °C. The reaction is controlled continuously by control analyses that monitor the loss of epoxidised oxygen. The reaction is completed when the epoxy bonds are completely saturated.

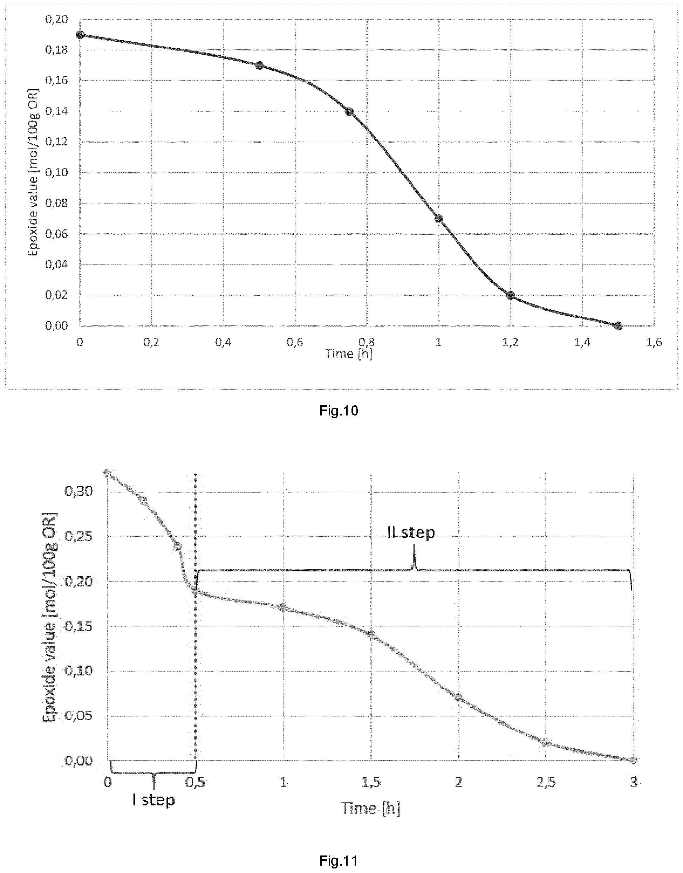

An example of the reaction progress in a one-step process is shown in **Fig.10**

The two-step process is carried out for an epoxidised oil with an epoxide number of 0.21-0.35 mole/100 g of epoxidised oil; an iodine number of less than 10 gI2/100g, preferably less than 5 gI2/100g, an acid number of less than 1 mgKOH/g, preferably 0.6 mgKOH/g. The first step of the epoxy ring-opening process occurs between the purified epoxidised oil and water in a molar ratio of 5 to 10 moles of water to moles of epoxy groups, preferably 6 moles of water to moles of epoxy groups, in the presence of an acid catalyst in the amount of from 2 to 5 g/l mole of epoxy groups, preferably from 2.5 to 3.5 g/ 1 mole.

In the process of opening epoxy groups, p-toluenesulfonic acid or sulphuric acid VI or tetrafluoroboric acid or phosphoric acid V is used as an acid catalyst.

An epoxidised oil having an epoxide number of 0,29 - 0,35 mol/100 g of epoxidised oil is introduced to the water with the catalyst. The whole is mixed at a stirring speed of 1000-1800 rpm, preferably not less than 1500 rpm, and heated to a reaction temperature in the temperature range of 60-100 °C, preferably 75+/- 5 °C. The whole is mixed at a stirring speed of 1000-1800 rpm, preferably not less than 1500 rpm, and heated to a reaction temperature in the temperature range of 60-100 °C, preferably 75+/- 5 °C. The reaction is carried out until an epoxide number in the range of 0.10-0.22 mol/100g of epoxidised oil is obtained, preferably 0.14-0.18 mol/100g, resulting in a 50% reduction of epoxide groups. The second step of epoxy ring-opening reaction takes place between the epoxidised groups remaining in the oil after the first step and a nucleophile at a molar ratio of 1-2 moles of nucleophile to moles of epoxy groups, preferably 1.2 moles of nucleophile to moles of epoxy groups and in the presence of an acid catalyst in an amount of from 2 to 5 g/l mole of epoxy groups, preferably from 2.5 to 3.5 g/ 1 mole.

Glycerol or glycols or alcohols or esters of higher fatty acids are used as nucleophiles.

Epoxidised oil having an epoxide number of 0.10-0.22 mol/100 g of epoxidised oil, preferably 0.14-18 mol/100g of epoxidised oil is introduced into the mixture of the nucleophile with the catalyst. The whole mixture is mixed at a stirring speed of 1000-1800 rpm, preferably not less than 1500 rpm, and the whole mixture is heated to the reaction temperature, but not less than 100 °C, preferably 120+/-5°C. The reaction is carried out until the epoxide groups, expressed as epoxide number, disappear. An example of the progress diagram of the two-step reaction is shown in Fig.11

The crude biopolyol, obtained by a one or two-step process, requires purification and neutralisation of the catalyst remaining after the epoxy ring opening process.

The following are used as neutralizers: 20% solution of sodium hydroxide or 30% sodium methanolate or sodium carbonate.

The amount of neutraliser is calculated in stoichiometric ratio to the catalyst. The stoichiometric ratio of catalyst to neutraliser is 1:1 mol/mol.

Water in an amount of 20 - 50% m/m, preferably 30% m/m, relative to the weight of the crude biopolyol obtained, together with the neutraliser, is added to the crude biopolyol at a temperature of 60 - 80°C, preferably 80°C, and the entire reaction mixture is stirred for 30 - 60 min, preferably 60 min, to fully neutralise the acid components. After completion of the neutralization process, in order to remove the neutralized catalyst, the obtained biopolyol is rinsed with water in an amount of 20 to 50% m/m in relation to the mass of the biopolyol, preferably 50% m/m. The rinsing operation is repeated until an acid number of 0.4-1.2 mgKOH/g is obtained, preferably below 1 mgKOH/g.

The plant biopolyol thus prepared is again subjected to a distillation process using saturated steam at atmospheric pressure. As a result of this process, a biopolyol is obtained with a water content in the range of 20-60% m/m, and a by-product in the form of condensate, containing volatile organic compounds. The biopolyol distillation process is carried out until an acid number below 1.2 mgKOH/g is obtained, preferably below 1 mgKOH/g.

The final step in the production of biopolyol according to the invention is the removal of water from the produced biopolyol, using vacuum distillation. The tests have revealed that biopolyols with the best properties are obtained by vacuum distillation at a temperature of 80°C - 120°C, preferably 100+/- 2°C, and under vacuum conditions of 0.5 - 1.0 mbar, until a water content below 0.1% m/m, preferably below 0.07% m/m, is achieved.

The biopolyols obtained are characterised by the following properties:
- iodine number of less than 10 gI2/100g, more preferably less than 5 mgI2/100g, indicating that the double bonds have completely reacted,
- acid number of less than 2 mgKOH/g, preferably less than 0,8 mgKOH/g,
- hydroxyl number in the range 80 - 320 mgKOH/g, most preferably close to 125 mgKOH/g.

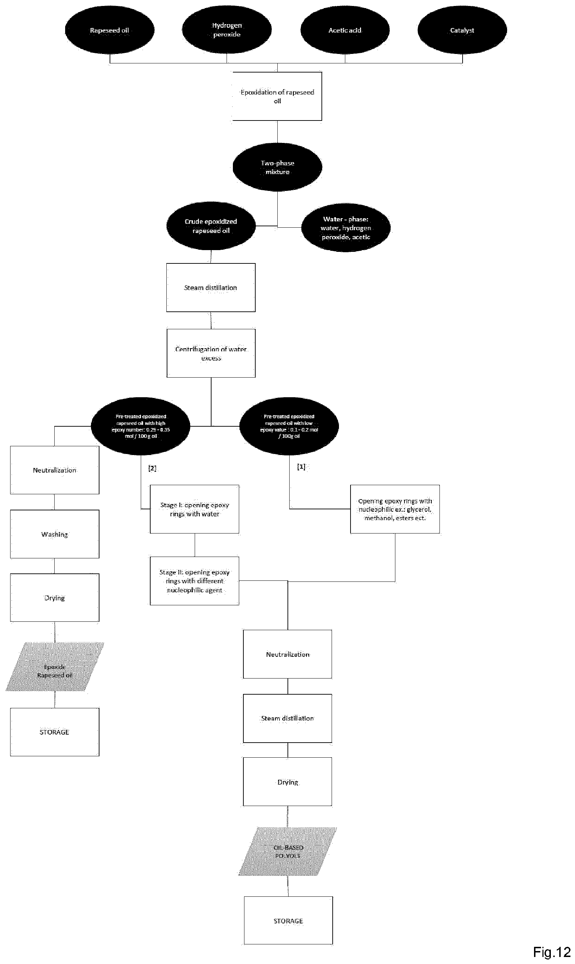

A diagram of the technological process is presented in Fig. 12

The use of biopolyol, produced according to the invention, allows its use in 100% as a polyol component in the production of polyurethanes, without the use of petrochemical polyols in the mixture, which will significantly reduce the production of greenhouse gases in the form of CO2 in relation to the previously produced petrochemical polyols.

### EXAMPLES

The object of the invention is presented in examples of implementation, without limiting other possibilities of implementation.

### Example 1

A glass reactor with a capacity of 10 L equipped with a reflux condenser, a component dosing system, a thermometer and a mechanical stirrer, was filled with 3000 g of degummed rapeseed oil with the following parameters:
- iodine number 110 gI2/100g,
- acid number 0,05 mgKOH/g,
- phosphorus content 15ppm,
- water content 0,0600 % by weight .

Subsequently, 534.2g of glacial acetic acid, and an acid catalyst in the form of 75% phosphoric acid solution, in the amount of 112.1g, were added. The contents of the reactor were heated to 62 °C, via a temperature-controlled water bath. When the contents of the reactor reached a temperature of 35 °C, the dosage of 3304 g of 35% dihydrogen peroxide was started, with the rate of temperature increase of 4°C /h. All of the dihydrogen peroxide was introduced before the temperature reached 55 °C, after which heating of the entire reaction mixture was continued until the reaction temperature was reached. The epoxidation reaction lasted 16h. At the end of the reaction, the stirrer was turned off and the reactor contents cooled to room temperature. The epoxidation reaction resulted in a two-phase mixture, where the oil phase contains epoxide, the aqueous phase consists of water, acetic acid, unreacted perhydrol and catalyst. The mixture was separated in a 2000ml pear-shaped separator. The crude epoxidised oil was steam distilled and then centrifuged for 5min using a centrifuge at 5000rpm. For further storage of the epoxidised oil, neutralisation was carried out using a 30% sodium methanolate solution in the quantity of 628g. The epoxidised oil so neutralised was rinsed five times with hot water, after each rinse the mixture was separated in a pear-shaped separator with a tap with a volume of 2000ml tap. Subsequently, the epoxidised oil was dried using vacuum distillation at 120°C, with a vacuum of 0.8 mbar for 2h. The resulting epoxidised oil has the following parameters:
- iodine number 3 gI2/100g,
- acid number 0,3 mgKOH/g,
- epoxy oxygen content 5.72 % w/w.
- epoxy value 0,36 mol/100g
- water content 0,07 % by weight.

### Example 2

Proceeding as in Example 1, the same quantities of raw materials and equipment were used, the change is the catalyst. The process used hydrochloric acid 30% as a catalyst in an amount of 250g. The reactions were carried out at a temperature not exceeding 50 °C

The resulting epoxidised oil has the following parameters:
- iodine number 7 gI2/100g,
- acid number 0,9 mgKOH/g,
- epoxy oxygen content 5.23 % w/w.
- epoxy value 0,32 mol/100g
- water content 0,07 % by weight.

### Example 3

A glass reactor with a capacity of 10 L equipped with a reflux condenser, a component dosing system, a thermometer and a mechanical stirrer, was filled with 3000 g of degummed rapeseed oil with the following parameters:
- iodine number 107 gI2/100g,
- acid number 0,06 mgKOH/g,
- phosphorus content 17ppm,
- water content 0,07 % by weight.

341.8g of glacial acetic acid and acid catalyst in the form of 75% phosphoric acid solution in the amount of 98.2 g were added to the reactor. Using a water bath, the reactor with the contents was heated to 62 °C. When the temperature in the reactor reached 35 °C, the dosage of 2090 g of dihydrogen peroxide with a concentration of 35% was started at the rate of temperature increase of 4 °C /h. The epoxidation reaction was carried out at 62 °C, stirring the whole with a mechanical stirrer at 1800rpm for 12h. At the end of the reaction, the stirrer was turned off and the reactor contents cooled to room temperature. The epoxidation reaction yielded a two-phase mixture where the oil phase contains epoxidised oil and the aqueous phase is water, acetic acid, unreacted perhydrol and catalyst. The mixture was separated in a 2000ml pear-shaped separator. The crude epoxidised oil was steam distilled and then centrifuged using a laboratory centrifuge at 5000rpm for 5min. The epoxidised oil thus obtained was used immediately for the production of biopolyol, i.e. in the ring-opening process. Properties of the obtained oil:
- iodine number 5 gI2/100g,
- acid number 0,8 mgKOH/g,
- epoxy oxygen content 5.23 % w/w.
- epoxy value 0,35 mol/100g
- water content 3 % by weight.

### Example 4

A glass reactor with a capacity of 10 L equipped with a reflux condenser, a component dosing system, a thermometer and a mechanical stirrer, was filled with 3250 g of degummed rapeseed oil with the following parameters:
- iodine number 109 gI2/100g,
- acid number 0,05 mgKOH/g,
- phosphorus content 13ppm,
- water content 0,07 % by weight.

Subsequently, 377.2g of glacial acetic acid, and 107.3g of 75% phosphoric acid catalyst were added. Using a water bath, the reactor contents were heated to a reaction temperature of 62 °C. The dosing of 2305 g of dihydrogen peroxide with a concentration of 35% was started when the temperature in the reactor reached 35 °C , the dosing rate was according to the temperature increment of 4 °C /h. The epoxidation reaction was carried out at 62 °C by stirring the whole with a mechanical stirrer at 1800rpm for 18h in order to prolong the reaction to use the product in the one-step epoxy ring opening process. At the end of the reaction, the stirrer was turned off and the reactor contents cooled to room temperature. The epoxidation reaction resulted in a two-phase mixture, where the oil phase contains epoxide, the aqueous phase consists of water, acetic acid, unreacted perhydrol and catalyst. The mixture was separated in a 2000ml pear-shaped separator. The crude epoxy was steam distilled and then centrifuged using a laboratory centrifuge at 5000rpm for 5min. The resulting epoxidised oil was used immediately in a one-step epoxy ring opening process.

Parameters of the obtained epoxidised oil:
- iodine number 0 gI2/100g,
- acid number 0,9 mgKOH/g,
- epoxy oxygen content 2.89 % w/w.
- epoxy value 0,18 mol/100g
- water content 3,5 % by weight.

### Example 5

A glass reactor with a capacity of 10 L equipped with a reflux condenser, a component dosing system, a thermometer and a mechanical stirrer, was filled with 298.4 g of glycerine in an amount of 298,4 g and tetrafluoroboric acid with a concentration of 48% in an amount of 17,44g. Heating of the reactor contents to the reaction temperature i.e. 120 °C in a glycerine bath was started. During heating, the mixture of glycerine and catalyst was supplemented with 1500 g of purified epoxidised oil with the following parameters :
- iodine number 0 gI2/100g,
- acid number 0,9 mgKOH/g,
- epoxy oxygen content 2.89 % w/w.
- epoxy value 0,18 mol/100g
- water content 3,5 % by weight.

The ring-opening reaction was carried out at 120 °C, stirring the whole with a mechanical stirrer at 1750 rpm for 100min. After the reaction was completed, the crude biopolyol was neutralized by introducing 500g of water at 80 °C into the reactor along with 21g of sodium methanolate at 30% concentration. The entire reaction mixture, together with the neutraliser and water, was stirred at 80 °C, for 40 min. After neutralisation, the biopolyol was rinsed six times with water in the amount of 700g each time. After neutralisation and washing, the biopolyol was subjected to steam distillation to remove volatile products. After the steam distillation process, the product contained 30% water, so the product was vacuum distilled at 100 °C and reduced pressure of 0.8mbar to remove the water.

The biopolyols obtained are characterised by the following properties:
- iodine number 0 gI2/100g
- acid number 0,8 mgKOH/g
- acid number 125 mgKOH/g
- water content 0,056

### Example 6

Proceeding as in Example 5, the same quantities of raw materials and equipment were used, the change is the catalyst and nucleophile. The process used phosphoric acid V 75% as catalyst and methanol as epoxy ring opener. The amount of catalyst used is 12g, and the amount of nucleophile used is 518g. The reactions were carried out at a temperature not exceeding 53 °C. The resulting epoxidised biopolyol has the following parameters:
- iodine number 2 gI2/100g
- acid number 0,7 mgKOH/g
- acid number 110 mgKOH/g
- water content 0.06 %

### Example 7

A glass reactor with a capacity of 10 L equipped with a reflux condenser, a component dosing system, a thermometer and a mechanical stirrer, was filled with water in the amount of 162g and tetrafluoroboric acid with a concentration of 48% in an amount of 10,34g. Heating of the reactor contents to the reaction temperature i.e. 80 °C in a glycerine bath was started. During heating, the mixture of water and catalyst was supplemented with 1000 g of epoxidised oil with the following parameters :
- iodine number 5 gI2/100g,
- acid number 0,8 mgKOH/g,
- epoxy oxygen content 5.23 % w/w.
- epoxy value 0,35 mol/100g
- water content 3 % by weight.

The first step of ring-opening reaction was carried out at 80 °C, stirring the whole with a mechanical stirrer at 1750 rpm for 30min. After the reaction was completed, the contents of the rector was removed, and 176.8g of glycerol and 10.33g of 48% tetrafluoroboric acid were then introduced therein. The reactor contents were heated to 120 °C; while heating, partially reacted epoxy oil - the product after the first step of the process - was introduced. The reaction was carried out for 90min at 120 °C, stirring the whole with a mechanical stirrer at 1800rpm. After the ring-opening process was completed, the crude biopolyol was neutralised by introducing 300g of water at 80 °C into the reactor along with 24.5g of sodium methanolate at 30% concentration. The entire reaction mixture along with the neutraliser and water was stirred with a mechanical stirrer at 1700 rpm at 80 °C for 60min. After neutralisation, the biopolyol was rinsed six times with water in the amount of 500g each time. After neutralisation and washing, it was subjected to steam distillation to remove volatile products. After the steam distillation process, the product contained 30% water, so the product was vacuum distilled at 100 °C and reduced pressure of 0.8mbar to remove the water.

The biopolyols obtained are characterised by the following properties:
- iodine number 2 gI2/100g
- acid number 0,5 mgKOH/g
- acid number 124 mgKOH/g
- water content 0,08

### Example 8

A glass reactor with a capacity of 1000 L equipped with a reflux condenser, a component dosing system, a thermometer and a heating/cooling jacket, was dosed with 350 kg of degummed rapeseed oil with the following parameters:
- iodine number 109 gI2/100g,
- acid number 0,05 mgKOH/g,
- phosphorus content 17ppm,
- water content 0,058 % by weight.

40.6 kg glacial acetic acid, and an acid catalyst in the form of a 75% solution of phosphoric acid in the amount of 11.6 kg were added to the reactor. The reactor contents were heated to a reaction temperature of 62 °C. When the contents of the reactor reached a temperature of 35 °C, the dosage of 248.2 kg of 35% dihydrogen peroxide was started, with the rate of temperature increase of 4°C /h. The epoxidation reaction lasted 15h. After completion of the reaction, the entire mixture was cooled to room temperature and the stirrer was turned off for phase separation. The aqueous phase was removed using a drain port. The crude epoxide was steam distilled by introducing a steam nozzle into the reactor. After the distillation process, the mixture was again separated and the water phase removed. The epoxide was centrifuged using a centrifugal separator. The resulting epoxidised oil has the following parameters:
- iodine number 7 gI2/100g,
- acid number 0,7 mgKOH/g,
- epoxy oxygen content 5.52 % w/w.
- epoxy value 0,34 mol/100g

### Example 9

A glass reactor with a capacity of 1000 L equipped with a reflux condenser, component dosing system, thermometer, mechanical stirrer and heating/cooling jacket was filled with water in the amount of 43.2 kg and tetrafluoroboric acid with a concentration of 48% in an amount of 2,75 kg. Heating of the reactor contents to the reaction temperature i.e. 80 °C was started. During heating, the mixture of water and catalyst was supplemented with 250 kg of epoxidised oil with the following parameters :
- iodine number 7 gI2/100g,
- acid number 0,7 mgKOH/g,
- epoxy oxygen content 5.52 % w/w.
- epoxy value 0,34 mol/100g

The first step of ring-opening reaction was carried out at 80 °C, stirring the whole with a mechanical stirrer at 1750 rpm for 30min. The reactor was emptied of epoxide prereacted to the epoxide number of 0.18 mol/100 g oil. 49.75 kg of glycerine and 2.9 kg of 48% tetrafluoroboric acid were then introduced into the reactor. The reactor contents were heated to 120 °C , while heating, partially reacted epoxy oil - the product after the first step of the process - was introduced. The reaction was carried out for 90min at 120 °C, stirring the whole with a mechanical stirrer at 1800rpm. After the ring-opening process was completed, the crude biopolyol was neutralised by introducing 200kg of water at 80 °C into the reactor together with 30% sodium methanolate in an amount of 6.7kg.. The entire reaction mixture, together with the neutraliser and water, was stirred with a mechanical stirrer at 1700 rpm at the temperature of 80 °C, for 60 min. After neutralisation, the biopolyol was rinsed six times with 100 kg of water, each time the excess water is removed through a drain connection. In the next stage, steam nozzles were introduced to distil the volatile products of the reaction. Then, the biopolyol was dried using vacuum distillation at 100 °C and reduced pressure of 0.8mbar for 2.5h.

The biopolyols obtained are characterised by the following properties:
- iodine number 6 gI2/100g
- acid number 0,9 mgKOH/g
- acid number 153 mgKOH/g
- water content 0,09

### Example 10

A cup test was performed to foam the biopolyol obtained in Example 7 with diphenylmethane diisocyanate (MDI). The 200ml cup was filled with measured 14.16g of biopolyol, 0.67g of crosslinking agent in the form of glycerol from the vegetable oil transesterification process, 0.14g of silicone surfactant, 0.11g of amine catalyst mixture in the form of PMDTA and N, N- dimethylcyclohexylamine. Water in the amount of 0.29 g was used as a foaming agent. The entire pre-mix was thoroughly mixed and heated to 32 °C, to which 12.6g of MDI was added and mixed again. The growth of the polyurethane foam in the cup was observed. An image showing the trial is fig. 13

## Claims

1. The method of producing epoxidised rapeseed oil using the process of double bond epoxidation in the presence of a catalyst, atmospheric steam distillation, and dehydration, neutralisation and finally vacuum distillation, where the rapeseed oil feedstock has previously undergone pre-treatment comprising hot pressing, solvent extraction, degumming and neutralisation with phosphoric acid and deacidification with soda lye, **characterised in that** the rapeseed oilhaving a free fatty acid content of 0.02 to 0.1 % m/m; a phosphorus content of 5 to 100 ppm; a peroxide number of not more than 5 mqO2/kg; an alkalinity of 10 to 100 ppm; a water content of not more than 0.1 % m/m; an iodine number of 105 to 120 gI2/100g; an unsaponifiable matter content of not more than 1,5 % m/m, and a content of monoacylglycerols, diacylglycerols and free glycerol totalling not more than 1 % m/m, are placed in a reactor with stirrer and subjected to: [1] the process of epoxidation of double bonds using an oxidation system consisting of dihydrogen peroxide 35% and glacial acetic acid 99% in the presence of an acid catalyst, where the content of dihydrogen peroxide 35% to each mole of double bonds of rapeseed oil being from 1.0 to 5.0 moles, the content of glacial acetic acid 99% to each mole of rapeseed oil double bonds is from 0.1 to 1.0 mole, and the content of acid catalyst is from 1.0 to 6.0g m/m of concentrated catalyst per 100g m/m of reaction mixture, and the epoxidation process is carried out at stirrer speed from 800 to 1800 rpm at a temperature of 35 to 75°C with an epoxidation reaction selectivity of more than 70% and an epoxidation reaction conversion of more than 90%; [2] purification by known methods of residual dihydrogen peroxide 35% and acetic acid 99%; [3] distillation under atmospheric pressure using saturated steam to achieve an acid number below 2 mg KOH/g, preferably below 1 mg KOH/g, and [4] removal of excess water by centrifugation; [5] neutralization using a 30% sodium methanolate solution in relation to the amount of catalyst used in the epoxidation process 3: 1 mol/mol; [6] washing the oil several times with hot water; [7] separation of the resulting mixture by a known method; [8] drying by vacuum distillation at 80-120°C using a vacuum of 0.6 - 0.8 mbar until a water content of less than 0.1% m/m, preferably 0.06% m/m, is obtained; and [9] storing the epoxidised rapeseed oil for further processing as required

2. The method according to claim 1, **characterised in that** inorganic acids, preferably sulphuric acid VI or phosphoric acid V or hydrochloric acid, are used as an acid catalyst.

3. The method according to claim 1 or 2, **characterised in that** the double bond epoxidation process is carried out using an oxidation system comprising dihydrogen peroxide 35%, whose content for each mole of double bonds of the rapeseed oil is in the range of 1.5 to 2.0 moles, and glacial acetic acid 99%, whose content for each mole of double bonds of the rapeseed oil is in the range of 2.0 to 3.5g.m/m.

4. The method according to any of claims 1 to 3, **characterized in that** the epoxidation process, is carried out at a stirring speed of 1500 rpm at a temperature of 63°C, with an epoxidation reaction selectivity of more than 75% and an epoxidation reaction conversion of more than 95%.

5. The method according to any of claims 1 to 4, **characterised in that** for obtaining an epoxidised oil with an epoxide number of 0.21-0.35 mole/100 g of epoxidised oil, the epoxidation process is carried out over a time period of from 5 to 16h, preferably 9h, and for obtaining an epoxidised oil with an epoxide number of 0.1-0.2 mole/100 g of epoxidised oil, the epoxidation process is carried out over a time period of from 16 to 20h, preferably 17-18h.

6. The method according to any of claims 1 to 5, **characterized in that** the acid catalyst and glacial acetic acid 99% are added to the reactor as one portion, and successively dihydrogen peroxide 35% is introduced into the reactor at a temperature of 35 to 55 °C, preferably 45 °C by gradually dropping it at a constant rate of 1 to 10 °C /1h, preferably 4 °C /1h, with a constant temperature rise, max. to 55 °C, and after all components have been injected, the reaction mixture is gradually heated to a reaction temperature of 35 to 75 °C, preferably 57 to 63 °C.

7. The method according to any of claims 1 to 6, **characterised in that**, after a steam distillation process, the excess water in the purified epoxidised rapeseed oil is centrifuged to a water content of no more than 5% m/m, preferably no more than 2% m/m.

8. The method according to any of claims 1 to 7, **characterised in that** the vacuum distillation process is carried out at 120°C under a vacuum of 0.8 mbar.

9. The method according to any of claims 1 to 8, **characterised in that** the purified and dried epoxidised rapeseed oil has: an epoxidised oxygen content of from 4.0 to 6.0% m/m, preferably above 5.0% m/m; an iodine number of less than 10gI2/100g, preferably less than 5 gI2/100g; an acid number of less than 1.0 mgKOH/g, preferably from 0.7 to 1.0 mgKOH/g.

10. Method of producing a biopolyol using purified epoxidised rapeseed oil produced according to claims 1 to 7 with a water content of 2 - 5% m/m or according to claims 1 to 9 with a water content of less than 1% m/m, **characterised in that** the epoxidised rapeseed oil is subjected successively [1] to an epoxy ring-opening process with a nucleophile in the presence of an acid catalyst, wherein the purified epoxidised oil having an epoxide number of 0.1 to 0.2 mol/100 gram of epoxidised oil is subjected to a one-step epoxide ring-opening reaction with nucleophile at a ratio of 1-2 moles of nucleophile to moles of epoxide groups of the purified epoxidised oil, in the presence of an acid catalyst in the amount of 2 to 5 g/1 mol of epoxide groups, and the purified epoxidised oil having an epoxide number from 0.29 to 0.35 mole/100 g of epoxidised oil is subjected to a two-step epoxy ring-opening reaction, wherein the first step the reaction takes place in the presence of water having a molar ratio of 5 to 10 mole, preferably 6 mole of water to moles of epoxide groups, the acid catalyst contribution being from 2 to 5 g/1 mole of epoxide groups, and the reaction is carried out until an epoxide number of 0.10 to 0.22 mol/100g of epoxidised oil is obtained, whereas in the second step, the reaction is carried out between the remaining epoxidised groups in the oil obtained in the first step and a nucleophile at a molar ratio of 1-2 moles of nucleophile to moles of epoxide groups and with the participation of an acid catalyst in the amount of 2 to 5 g/1 mol of epoxide groups; and the crude biopolyol thus obtained is subjected successively [2] to catalyst naturalization process; [3] to rinsing with water to obtain an acid number from 0.4 to 1.2 mgKOH/g; [4] distillation process using saturated steam at atmospheric pressure until an acid number below 2 mgKOH/g is obtained; [5] vacuum distillation process at a temperature from 80°C to 120°C, preferably 100+/- 2°C and under negative pressure from 0.5 to 1.0 mbar.

11. The method according to claim 10, **characterized in that** the epoxidised oil with a water content of 2 to 5% m/m, produced according to claims 1 to 7 is transferred to the epoxy ring-opening process immediately after completion of the centrifugation process

12. The method according to claim 10 or 11, **characterized in that** the epoxy ring opening process uses glycols or glycerol or methanol or fatty acid esters or water as nucleophiles.

13. The method according to any of claims 10 to 12, **characterised in that** the epoxy ring-opening process uses as catalyst p-toluenesulphonic acid or sulphuric acid VI or tetrafluoroboric acid or phosphoric acid V in a preferable amount of 2.5 to 3.5 g/ 1mol of epoxy groups of the purified epoxidised oil.

14. The method according to any of claims 10 to 13, **characterised in that** the one-step epoxy ring-opening reaction process is carried out in a reactor with stirrer and full metering, into which a mixture of catalyst and nucleophile is introduced, followed by the addition of purified epoxy oil and the whole is stirred at a speed of 1000 to 1800 rpm, preferably not less than 1500 rpm while heating to a reaction temperature of not less than 100 °C, preferably 120+/- 5 °C, and the process is carried out to until the epoxy bonds are completely saturated.

15. The method according to any of claims 10 to 14, **characterized in that** the process of the two-step epoxy ring-opening reaction is carried out in a reactor provided with a stirrer, wherein after the introduction of the purified epoxidised oil with water in the presence of an acid catalyst, the first step of the process is carried out and the introduced products are mixed at a stirrer speed of from 1000 to 1800 rpm, preferably not less than 1500 rpm, and heated to a reaction temperature of 60 to 100°C, preferably 75 +/- 5°C, and the reaction is carried out until an epoxide number in the range of 0.10 to 0.22 mol/100g of epoxidised oil, preferably 0.14 to 0.18 mol/100g of epoxidised oil is obtained, after which the epoxidised oil obtained in the first step is introduced into the mixture of the nucleophile with the catalyst and the second step of the process is started by stirring the mixture at a stirring speed of 1000 to 1800 rpm, preferably not less than 1500 rpm, and heating to a reaction temperature of not less than 100 °C, preferably 120+/- 5 °C, and the reaction is carried out until the epoxy groups expressed as epoxide number disappear.

16. The method according to any of claims 10 to 15, **characterized in that** the neutralization process consists **in that**, at a temperature of 60 to 80°C, preferably 80°C, the reactor containing the crude biopolyol and the catalyst is supplemented respectively with: the neutralizer in a stoichiometric ratio to the catalyst of 1: 1 mol/mol and water in an amount from 20 to 50% m/m, preferably 30% m/m, relative to the weight of the crude biopolyol with the neutralizer and the entire reaction mixture is stirred for 30 to 60 min, preferably 60 min, to fully neutralize the acid components

17. The method according to claim 16, **characterized in that** as neutralizer one uses: 20% solution of sodium hydroxide or 30% sodium methanolate or sodium carbonate.

18. The method according to any of claims 10 to 17, **characterised in that**, after the neutralisation process, the biopolyol is rinsed several times with water in an amount of 20 to 50% m/m relative to the weight of the biopolyol, preferably 50% m/m, and the rinsing operation is repeated until an acid number of 0.4-1.2 mgKOH/g is obtained, preferably less than 1 mgKOH/g.

19. The method according to any of claims 10 to 18, **characterized in that**, the atmospheric distillation process of the biopolyol is carried out until an acid number below 1 mgKOH/g is obtained.

20. The method according to any of claims 10 to 19, **characterized in that** the biopolyol obtained has the following properties: water content less than 0.1% m/m, preferably less than 0.07% m/m; iodine number less than 10 gI2/100g, preferably less than 5 mgI2/100 g; acid number less than 2 mgKOH/g preferably less than 0.8 mgKOH/g; hydroxyl number 80 - 320 mgKOH/g, preferably 120-140 mgKOH/g.
